# EUROPEAN PATENT APPLICATION

(11) **EP 4 455 296 A1**
(43) Date of publication of application: **30.10.2024**
(21) Application number: 22910264.5
(22) Date of filing: 16.12.2022
(51) Int. Cl.: C12P 7/625, C12P 7/6445, C12P 7/6463, C08G 63/06, C08G 63/78, C02F 3/12

(54) **METHOD FOR PRODUCING POLYHYDROXYALKANOATES (PHAS) AND TRIACYLGLYCERIDES (TAGS)**

(30) Priority: 22.12.2021 ES 202131193
(71) Applicant: Universidade de Santiago de Compostela, 15782 Santiago de Compostela, A Coruña (ES)
(72) Inventor: MOSQUERA CORRAL, Anuska, 15782 Santiago de Compostela (A CORUÑA) (ES); VAL DEL RIO, Maria Angeles, 15782 Santiago de Compostela (A CORUÑA) (ES); ARGIZ MONTES, Lucia, 15782 Santiago de Compostela (A CORUÑA) (ES)
(74) Representative: Elion IP, S.L.
(86) International application number: PCT/ES2022/070806
(87) International publication number: WO 2023/118630

(57) **Abstract**

Disclosed is a single-unit biotechnology method for simultaneously recovering polyhydroxyalkanoates (PHAs) and triacylglycerides (TAGs). The present invention relates to a method for producing biomass in the form of a mixed microbial culture containing PHAs and TAGs. More specifically, the method comprises certain steps that occur in a single discontinuous sequential reactor.

## Description

### Field of the invention

The present invention relates to a biotechnological process for producing biomass containing intracellular PHA and TAG. More particularly, this process comprises certain steps that take place in a single sequencing batch reactor.

### Background

Only certain microorganisms are capable of synthesising and intracellularly accumulating polyhydroxyalkanoates (PHA) and triacylglycerides (TAG) under stress conditions (Carsanba et al., Crit. Rev. Biotechnol. 2018, 38, 1230-1243). To that end, when working with mixed microbial cultures (MMC), which mixture incorporates, among others, microorganisms capable of intracellularly accumulating PHA and microorganisms capable of intracellularly accumulating TAG, it is necessary to carry out a step of **enrichment** to select those populations with the greatest capacity to accumulate these compounds.

To that end, Sequencing Batch Reactor (SBR) type reactors are usually used. There are various strategies to carry out said selection process, although the most common is the so-called Aerobic Dynamic Feeding (ADF) strategy. This is based on the application of feast/famine cycles in aerobic conditions in which the culture is subjected to short periods of excess (feast) and long periods of deficiency of carbon source (famine) (Kourmentza et al., Bioengineering, 2017, 4, 55). In this regard, the microorganisms with the capacity to accumulate the substrate during the feast phase have a competitive advantage over the rest during the famine phase, since they can use intracellular carbon as a source of matter and energy for their growth. However, non-accumulators spend a long period of time without substrate and cannot duplicate themselves in such a way that after the application of successive feast/famine cycles they are washed out of the system (Valentino et al., N. Biotechnol. 2017, 37, 9-23).

In the last decade, different variations of the ADF strategy were proposed to optimise culture enrichment. Among them, the decoupling of the feeding from carbon and nitrogen sources stands out, which is known as Double Growth Limitation (DGL) strategy. By adding excess carbon during the feast phase under limiting nitrogen conditions, the growth of non-accumulating populations is prevented. Only those microorganisms capable of metabolising the substrate and accumulating it intracellularly will be able to consume the carbon source. Therefore, by adding nitrogen at the end of the feast phase (in the absence of carbon, since it was consumed during feast) the populations that accumulated PHA and/or TAG during the feast phase can use the nitrogen for growth, resulting in a faster and more efficient selection of the MMC (Kourmentza et al., Bioengineering, 2017, 4, 55).

Subsequently, the biomass enriched in the SBR is used as an inoculum in a second unit, which is a Fed Batch Reactor (FBR) type **accumulation** reactor in which the substrate is available in excess to maximise the amount of intracellular compounds accumulated before their extraction and purification. It involves exposing the biomass to an extended feast phase in aerobic conditions and absence of nitrogen as long as the substrate allows it, since this forces the conversion of the substrate to PHA and/or TAG, preventing the growth of non-accumulating populations. The preferred feeding form in systems of this type is the pulse feeding strategy, since it allows high accumulation efficiencies to be obtained, avoiding the effect of substrate inhibition. This consists of adding successive pulses of carbon once the previous one has been consumed until the maximum accumulation capacity is reached (Kourmentza et al., Bioengineering, 2017, 4, 55; Mannina et al., Bioresour. Technol. 2020, 297, 122478). The most common way to know when the substrate should be added is to monitor the concentration of dissolved oxygen (DO) in the system. After the addition of a carbon pulse the DO decreases, which indicates that there is substrate consumption, and returns to saturation values once this has been exhausted. At this point, a new pulse is added, and so on until, after the addition of the carbon source, a decrease in the DO concentration is observed, indicating that the maximum accumulation capacity of the MMC was reached.

In addition to these two reactors, when streams containing organic compounds that are difficult to metabolise are used, as is the case of lipids, a step of **substrate pretreatment** is performed in a third reactor to obtain soluble organic acids that are easier to metabolise (normally volatile fatty acids (VFA), the most commonly used precursors in the synthesis of PHA). It is generally an acidogenic fermentation process carried out in a Continuous Stirred Tank Reactor (CSTR) (Mannina et al., Bioresour. Technol. 2020, 297, 122478).

Up until now, obtaining intracellular compounds with high added value from lipids focussed on the use of pure microbial cultures and plant oils as substrate (Basnett et al., J. Mater. Sci. Mater. Med. 2018, 29.; Donot et al., Biomass and Bioenergy 2014, 68, 135-150; Pérez-Arauz et al., Food Packag. Shelf Life 2019, 20, 100297). However, these processes are not competitive on a large scale due to their high costs. To that end, in recent years, research was aimed at the development of processes based on the use of MMC that do not require sterile conditions and allow the use of waste streams as a substrate (De Donno Novelli et al., Bioresour. Technol. 2021, 331, 124985).

For now, existing studies related to the production of PHA and/or TAG with mixed culture and from waste streams are very scarce.

On the one hand, Campanari et al. (Int. J. Biol. Macromol. 2014, 71, 34-41) demonstrated the possibility of producing PHA from wastewater generated in the production of olive oil by using a system with three steps in series carried out in several reactors: pretreatment, enrichment and accumulation. The pretreatment process consisted of two phases: 1) solid phase extraction for the removal and recovery of polyphenols; and 2) anaerobic acidification of the dephenolised wastewater for the conversion of the remaining organic matter into volatile fatty acids (VFA), precursors in the synthesis of PHA only. The enrichment of the MMC, was carried out in an SBR-type reactor fed with pretreated wastewater rich in VFA. Subsequently, the biomass enriched in the SBR, was used as an inoculum in an FBR-type accumulation reactor, also fed with said pretreated waste stream.

Moreover, Tamis et al. (Biotechnol. Biofuels 2015, 8, 1-11) demonstrated the possibility of recovering TAG from wastewater using a MMC fed with a plant oil-water emulsion as a model substrate. To do this, they considered a system consisting of two sequential steps using two reactors: enrichment and accumulation carried out in SBR and FBR type reactors, respectively. In the first, the MMC with the capacity to accumulate TAG was selected, and subsequently, with the biomass enriched in this first step, the FBR reactor was inoculated to maximise the amount of TAG accumulated.

Lastly, the authors of the present invention were able to produce mostly PHAs or mostly TAGs from a fatty effluent generated in the canning industry. To that end, a process was used that comprised two reactors, an SBR-type enrichment unit, and another of FBR-type accumulation unit (Argiz et al., Sci. Total Environ. 2021, 763, 142944).

Therefore, it is still necessary to achieve a simpler and cheaper method that allows TAG, PHA or both simultaneously, to be obtained, avoiding pretreatment of readily available streams, preferably waste lipidic streams.

### Brief description of the invention

The authors of the present invention have developed a single-unit biotechnological process for the recovery of polyhydroxyalkanoates (PHA) and triacylglycerides (TAG) from lipidic streams using biomass in the form of mixed microbial cultures (MCC). In particular, they have designed a method that favours the development of a MMC with the capacity for the simultaneous accumulation of PHA and TAG intracellularly, using a simple process that is easy to implement in the industry.

An additional advantage is that this method is carried out in a single reactor, such that the installation of the process for its operation requires less implantation surface than that necessary for the processes that are currently used and that require three reactors in series.

Another advantage of the present invention is that it avoids the need for prior pretreatment of the biomass feed source (substrate), which simplifies the process and reduces start-up and operation times.

Another advantage is that when carried out in a single unit, daily productivity (expressed as g PHA and TAG produced per day and per litre of reactor) increases even for lower intracellular accumulation percentages. This is because, in the present invention, by performing volume exchange in the system at the end of the feast phase, the stream obtained already contains the biomass with the accumulated biopolymers, that can now be extracted and purified. However, in the conventional process, it is necessary to operate a second reactor, which lengthens processing times, thereby reducing productivity.

Another favourable aspect is that by reducing the number of reaction units, investment costs are reduced. Operating costs are also expected to be lower. This is because, for example, the costs associated with energy consumption in the pretreatment and accumulation reactors would be avoided (stirring, aeration, pumping, etc.).

It is a simpler process than conventional processes from a conceptual point of view. This allows errors to be minimised and facilitates the training of non-specialised personnel for the operation thereof.

Compared to existing mixed culture systems, the present invention is the only one that allows: 1) using a lipidic stream as a substrate without the need to pretreat it to obtain VFA in an independent unit; 2) carrying out the conventional pretreatment steps (in this case only hydrolysis), enrichment and accumulation in the same unit, and 3) obtain two different types of intracellular compounds with high added value, PHA and/or TAG, from the same carbon source.

Therefore, one aspect the present invention relates to a process for the intracellular production of polyhydroxyalkanoates (PHA) and triacylglycerides (TAG) in a mixed microbial culture, characterised in that all the steps are carried out in a single sequencing batch reactor, and which further comprises:
a) inoculating the reactor with activated sludge,
b) an operation cycle of the reactor that comprises:
   i. feeding the microorganisms present in the reactor with a lipidic carbon source, the feeding being in pulses and, under limiting nitrogen conditions,
   ii. removing 50% of the mixture from the reactor, wherein said mixture contains the biomass with intracellular PHA and TAG,
   iii. adding a stream containing between 0.9 and 2 times the stoichiometric amount of nitrogen necessary for microbial growth,
c) repeating the process from step b) in successive cycles.

### Description of the figures

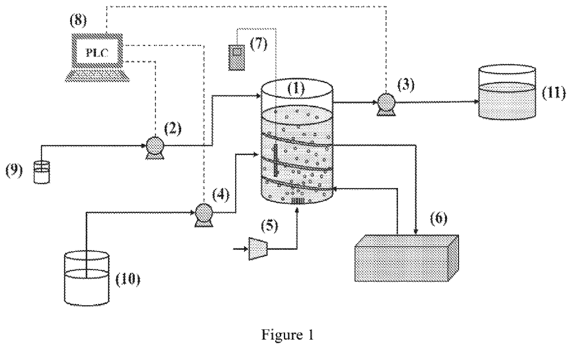

Figure 1 shows a simplified diagram of the single-unit process operated at laboratory scale for the recovery of PHA and TAG from a lipidic stream. (1) Sequencing Batch Reactor (SBR); (2) feed pump of the lipidic stream (carbon source); (3) discharge pump; (4) feed pump of the aqueous stream (nitrogen source); (5) blower; (6) thermostatted bath; (7) oxygen and temperature probe; (8) programmable logic controller (PLC); (9) storage of the lipidic substrate used as a carbon source (see composition in Table 1); (10) storage of the aqueous mixture containing the nitrogen source (see composition in Table 2); (11) storage of the reactor discharge containing the biomass with the accumulated intracellular compounds (PHA and TAG).

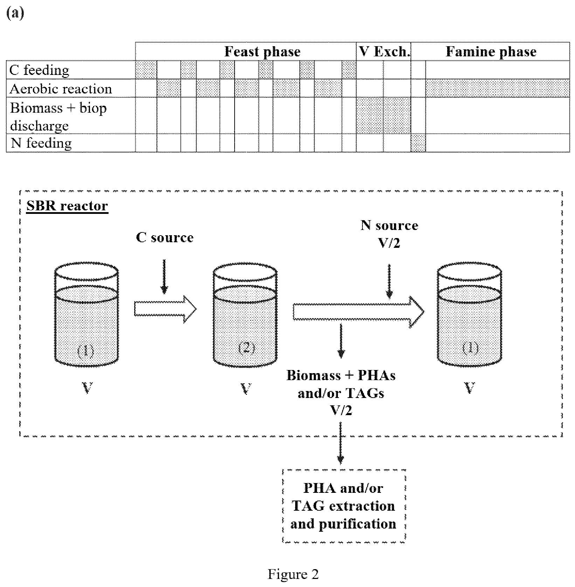

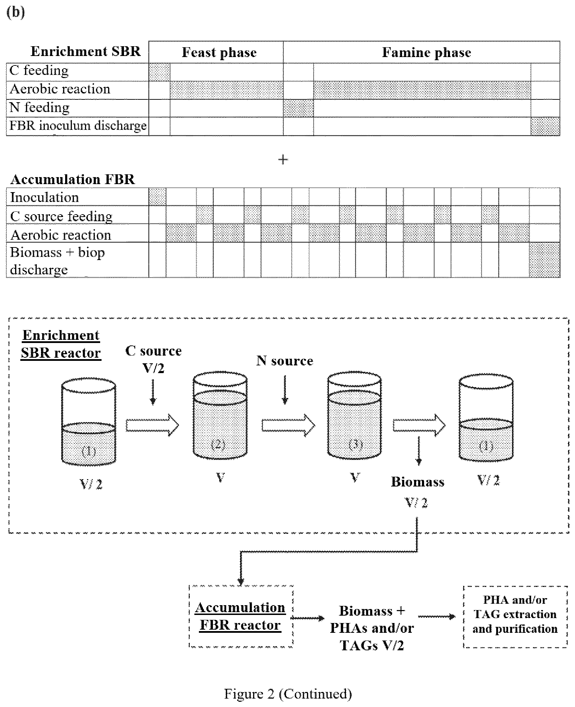

Figure 2 compares the configuration of the operation cycle of the SBR of the present invention (a) with that of a conventional SBR for enrichment and the subsequent accumulation step (b).

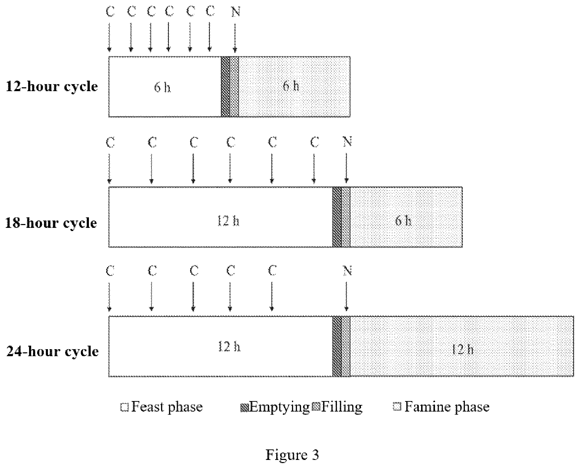

Figure 3 shows a simplified diagram of the different configurations of the operation cycle evaluated in the SBR reactor, wherein: C refers to the moment in which a pulse of lipidic substrate used as a carbon source is added; and N to the moment in which the aqueous stream containing the nitrogen source is added.

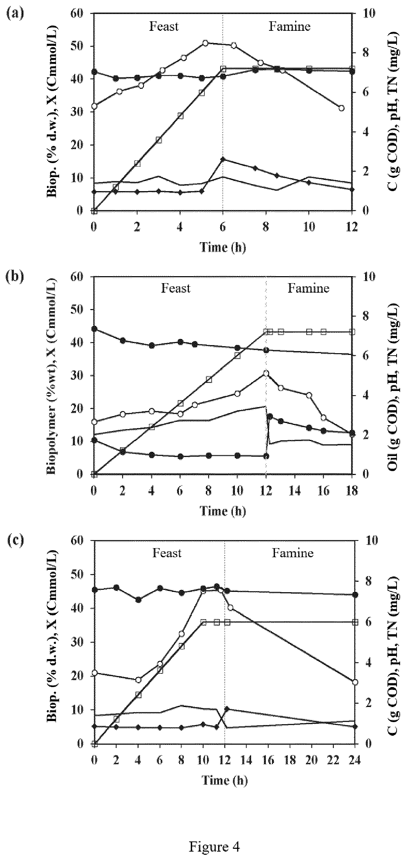

Figure 4 shows the complete characterisation of three operation cycles of the SBR reactor, (a) 12-hour cycle corresponding to day 43 of operation; (b) 18-hour cycle relating to day 72 of operation; and (c) 24-hour cycle referring to day 133 of operation. pH (•), X concentration (-), TN concentration (◆), % accumulated biopolymer (Biop) (o), and C concentration (□). Wherein: Biop refers to the sum of PHA and TAG accumulated intracellularly, said sum expressed as a percentage by dry weight of the cell (% d.w.); C to the cumulative amount of substrate added in pulses expressed in grams of chemical oxygen demand (COD); TN to the total nitrogen concentration in g/l; and X to the concentration, in Cmmol/L, of active biomass present in the reaction medium.

### Detailed description of the invention

In the present invention, **activated sludge** is understood as a flocculent mixture of microorganisms grown in an aeration tank under controlled conditions. They are used in biological wastewater treatment processes for the degradation of organic compounds.

In this invention, **mixed microbial culture** (MMC) is understood as that which is found in non-sterile conditions and consists of a mixture of species belonging to different groups of microorganisms (bacteria, fungi and yeast).

In the present invention, the term **biomass** refers to the set of microorganisms (bacteria, fungi and yeasts) initially present in the activated sludge used as an inoculum, and subsequently in the MMC enriched in populations with the capacity to hydrolyse the lipidic carbon source used as a substrate and accumulate PHA and TAG.

In the present invention, the expression **oleaginous microorganisms or oleaginous populations** refers to microorganisms or populations of microorganisms that are capable of accumulating more than 20% of their dry weight in triacylglycerides. Within the different classes of oleaginous microorganisms (microalgae, bacteria, yeasts and fungi), only certain species, especially yeasts, are capable of surviving in hydrophobic environments. This means they are capable of hydrolysing and metabolising lipidic carbon sources for intracellular accumulation. Some examples are oleaginous yeasts *Yarrowia*, *Cryptococcus*, *Rhodosporidium*, *Geotrichum* and *Trichosporon* (Patel and Matsakas, Ultrason. Sonochem. 2019, 52, 364-374).

In this invention the term **microbial activity** refers to the ability of heterotrophic microorganisms to decompose dissolved organic substances for the simultaneous formation of microbial biomass.

In the present invention, **substrate** is understood as the waste lipidic stream that contains the carbon source that the MMC uses to grow and accumulate PHA and TAG.

In the present invention, **lipidic carbon source** is understood as a stream with a lipid content greater than 85% of the COD, in particular between 85 and 90% COD, that microorganisms use as a carbon source for their growth.

In the present invention, **long chain fatty acids** is understood as those that are made up of chains of more than 14 carbon atoms. For example, oleic acid, linoleic acid, palmitic acid, stearic acid, etc. Preferably they have between 14 and 32 carbon atoms, more preferably between 14 and 26 carbon atoms.

In the present invention, **hydrolysis** is understood as the process by which the lipidic content of the substrate is solubilised in the aqueous phase to be available for transformation by microorganisms.

In the present invention, **enrichment** or MMC selection is understood as the process in which certain operational conditions (called selective pressures) are imposed that favour the growth of certain microorganisms initially present in the inoculum (activated sludge) in order to maximise their presence in the enriched MMC. The intention is to promote the survival of populations that are capable of carrying out certain functions, instead of specific microorganisms. The present invention promotes the growth of microorganisms capable of: 1) hydrolysing the lipidic stream used as a carbon source, and 2) metabolising the compounds resulting from hydrolysis (long chain fatty acids and glycerol) and accumulating them intracellularly as PHA and/or TAG.

In this invention, **accumulation** is understood as the process by which the synthesis of PHA and TAG is promoted inside the cells of the microorganisms and the amount of the same is increased inside the cells of the microorganisms present in the enriched MMC.

**Limiting nitrogen conditions** is understood as those in which nitrogen is found in a minimum amount in the reaction medium so that microbial growth is limited, in other words, in an amount less than 20 mg total nitrogen (TN)/L.

**Stoichiometric amount of nitrogen necessary for microbial growth** is understood as the moles of nitrogen required for the formation of one mole of biomass based on the elemental composition of the biomass that makes up the MMC (CH_{1.8}O_{0.5}N_{0.2}) (Popovic, Heliyon, 2019, 5, 6). Therefore, stoichiometrically, 0.2 moles of N are needed per mole of biomass present in the reaction medium.

In the present invention, the term **feast** refers to the period of reactor operation time in which the MMC has extracellular carbon available in excess in the reaction medium and under limiting nitrogen conditions.

In the present invention, **famine** is understood as the period of time in which the MMC does not have extracellular carbon available in excess in the reaction medium but does have nitrogen.

In the process of this invention, hydrolysis, enrichment and accumulation processes are carried out in a single unit, in other words, in a single reactor, which conventionally involve the operation of three reactors in series (CSTR + SBR + FBR).

In a preferred embodiment, the process is carried out in a single sequential reactor that operates in batch mode, of the Sequencing Batch Reactor (SBR) type.

The process of the invention comprises a first **step a)** which is characterised by inoculating activated sludge in a sequencing batch reactor.

In a preferred embodiment, the sludge of the present invention comprises microbial populations with the ability to perform the following functions:
1) Hydrolysing the fats present in the lipidic stream used as a carbon source into long chain fatty acids and glycerol.
2) Intracellularly accumulating the compounds resulting from the hydrolysis process such as PHA and TAG.

In a more preferred embodiment, the activated sludge of the invention comprises accumulating microorganisms. PHA-accumulating microorganisms can be, for example, *Acidovorax, Acmetobacter, Pandoraea, Azospirillum,* and *Pseudomonas*; TAG-accumulating microorganisms can be, for example, *Candida, Yarrowia, Geotrichum, Mortierella, and Rhodoccocus.*

In another preferred embodiment, the activated sludge of the invention comprises microorganisms capable of hydrolysing lipids. Generally, these microorganisms are oleaginous populations of fungi and yeasts, although some bacterial populations that have this function can also be found. These microorganisms capable of hydrolysing lipids can be, for example, *Candida, Yarrowia, Fonsecaea, Capronia, Acinetobacter, Cryptococcus, Rhodosporidium, Geotrichum* and *Trichosporon.*

In an even more preferred embodiment, the activated sludge of the invention comprises PHA- and TAG-accumulating microorganisms, and microorganisms capable of hydrolysing lipids.

The activated sludge used in the present invention can have different origins. In a preferred embodiment, the sludge used in the present invention is sludge from an urban Wastewater Treatment Plant (WWTP); in a more preferred embodiment, it is sludge from the aeration tank of the secondary treatment of a WWTP.

Another advantage of the present invention is that the culture is enriched in accumulating microorganisms and also in microorganisms that have the capacity to hydrolyse the lipidic stream used as a carbon source. Therefore, after adding the carbon source to the reaction medium, those microorganisms with the capacity to secrete extracellular lipases catalyse their hydrolysis, releasing long-chain fatty acids and glycerol (precursors in the synthesis of PHA and TAG).

In a preferred embodiment, the process of the invention does not comprise a pretreatment or hydrolysis step of the lipidic carbon source. In this way it is possible to directly use a lipidic stream without the need to use an additional reactor for pretreatment. It is not necessary to carry out a previous hydrolysis process to obtain long chain fatty acids and glycerol. Nor is it necessary to transform this stream into a more easily metabolisable carbon source such as volatile fatty acids (which can be obtained by acidification of a lipidic carbon source). This is possible due to the enrichment that is carried out in the process in which accumulating microorganisms and also microorganisms that allow lipids to be hydrolysed are selected. Another advantage of the process of the invention compared to the state of the art is that the configuration of the cycle of the reactor is designed in such a way that it allows, in a single reactor: i) selecting, from the activated sludge used as an inoculum, a culture with the capacity to hydrolyse the substrate and accumulate PHA and TAG; ii) reproducing the culture present in the reaction medium in each operation cycle; and iii) maximising the amount of accumulated PHA and TAG, i) and ii) are conventionally carried out in an SBR-type enrichment reactor; and iii) in a FBR-type accumulation reactor. Figure 2 shows a comparison between the configuration of the operation cycle of the SBR designed in the present invention (Figure 2 (a)) with that of an enrichment SBR and the subsequent accumulation step of the conventional process (Figure 2 (b)).

The process of the invention further comprises a step b) characterised by an operation cycle of the reactor.

This step b) comprises (i) feeding the microorganisms present in the reactor with a lipidic carbon source, the feeding being in pulses and under limiting nitrogen conditions, in other words, the amount of nitrogen is less than 20 mg total nitrogen (TN)/L. Preferably the amount of nitrogen is between 5 and 20 mg TN/L. More preferably, the amount of nitrogen is equal to or less than 5 mg TN/L.

This step (i) is a prolonged feast phase in which the carbon source is added in pulses over a long period of time and under limiting nitrogen conditions. The intention of this feeding strategy is for the amount of intracellular compounds accumulated at the end of the feast phase to be the maximum possible. This feast phase is designed so that the amount of intracellular compounds accumulated at the end of the feast phase is the maximum possible so that the biomass removed contains the PHA and TAG produced by the system.

The design of the feast phase in the invention avoids having to operate a second FBR-type accumulation reactor.

In the present invention, the feast phase has been designed to accumulate the maximum possible amount of biopolymers intracellularly (accumulation), so that the biomass obtained in the feast phase can now be treated to obtain biopolymers. Unlike conventional processes in which the feast phase is designed solely to feed the MMC to enrich it in the microorganisms of interest, and thus the accumulation to obtain the maximum amount of biopolymers possible must be carried out in another reactor, usually FBR type, which entails a greater number of steps and much longer processes than that of the present invention.

Therefore, to obtain the biomass with intracellular biopolymers ready for extraction and purification (> 30 - 40% by dry weight (d.w.) intracellular of PHA and TAG), having to inoculate an FBR-type reactor with the culture previously enriched in the SBR, and operating it by subjecting the microorganisms to a prolonged feast phase to maximise the amount of accumulated biopolymers is avoided in the present invention. Unlike what happens in the feast phase of a conventional SBR-type enrichment reactor, where it is only sought for the microorganisms with the capacity to metabolise the substrate to accumulate a certain amount of carbon that allows them to survive once the extracellular carbon is exhausted (famine phase), in the present invention, furthermore, the amount accumulated in the SBR is the maximum possible to avoid operating a second accumulation unit.

Therefore, during this first phase of the cycle (i), the pretreatment and accumulation processes take place simultaneously, which would traditionally be carried out in two reactors. In turn, this first phase contributes to the selection of the MMC. This is because only certain microorganisms will be able to accumulate PHA and TAG, and these will be the ones that during the famine phase will be able to use these intracellular compounds as a carbon source for growth and remain in the system.

The operation cycle of the reactor of step b) of the process of the invention, further comprises a step ii) in which 50% of the mixture is removed from the reactor, wherein said mixture contains the biomass with intracellular PHA and TAG.

An additional advantage of the present invention is that it obtains the biomass with PHA and TAG accumulated in an amount greater than 30% by intracellular dry weight (d.w.) of PHA and TAG, as shown in examples 1, 2 and 3, and thus can be taken directly to the subsequent PHA and TAG extraction and purification processes.

In a preferred embodiment, this step ii) takes place immediately after step i). This is a relevant difference with respect to what happens in conventional systems, in which the discharge of the biomass takes place at the end of the operation cycle of the SBR enrichment reactor, in other words, after the famine phase. In this way, after the famine phase, the microorganisms do not have accumulated intracellular compounds since they used them as a carbon source for growth. The biomass that is removed from the reactor after the famine phase is taken to an FBR-type reactor to be used as an inoculum in an accumulation process in which the aim is to maximise the amount of accumulated intracellular compounds. This is one of the reasons why conventional processes must use several reactors.

The operation cycle of the reactor of step b) of the process of the invention, further comprises a step iii) in which a stream containing between 0.9 and 2 times the stoichiometric amount of nitrogen necessary for microbial growth is added.

Therefore, in this step iii) the famine phase begins and the microorganisms will be without extracellular carbon in the liquid medium for a long period of time. Since there is no external carbon, only those microorganisms that accumulated PHA and TAG during the feast phase can use these compounds as a carbon source for growth. On the contrary, non-accumulating populations cannot replicate and will be washed out of the system. In this regard, by repeating several operation cycles, the system is progressively and naturally enriched in accumulating populations, the enrichment of the MMC taking place in this phase.

The amount of nitrogen added at the beginning of the famine phase should be, at most, the stoichiometric amount necessary for microbial growth. In a particular embodiment, the stream used in this phase is an aqueous solution with a nitrogen compound. In another particular embodiment, the nitrogen compound is selected from ammonium sulphate, ammonium nitrate, and ammonium chloride. In a more particular embodiment, the aqueous solution contains a concentration of between 5 mg/l and 280 mg/l of ammonium chloride.

In a preferred embodiment, at the end of phase (iii) the amount of total nitrogen in the reaction medium is less than 20 mg of total nitrogen per litre (< 20 mg TN/L), preferably less than 5 mg total nitrogen per litre (<5 mg TN/L). In this regard, in the next cycle in which the feast phase begins by feeding with the carbon source, as it takes place in the presence of a nitrogen level less than 20 mg of total nitrogen per litre (< 20 mg TN/L), preferably less than 5 mg total nitrogen per litre (< 5 mg TN/L), accumulation is favoured and the lipidic stream is prevented from being used as a carbon source for growth. The best results were obtained when step (i) took place under nitrogen concentrations close to 5 mg TN/L, as demonstrated in example 1. Therefore, preferably the nitrogen concentration at the end of phase (iii) is equal to or less than 5 mg TN/L and phase (i) takes place under a nitrogen concentration equal to or less than 5 mg TN/L.

The process of the invention further comprises a step c) which is the repetition of the process from step b) in successive cycles.

In a particular embodiment, the process of the invention comprises operation cycles of variable lengths with lengths of 12, 18 and 24 hours in different operational periods. In another particular embodiment, step (i), the feast phase lasts between 6 and 12 hours. In another particular embodiment, step (iii), the famine phase lasts between 6 and 12 hours.

The method of the present invention has the potential to allow biomass to be obtained that contains different proportions of PHA and TAG depending on the operating conditions and, therefore, the enrichment of the culture in one microorganism or another.

In a particular embodiment, the process of this invention allows the intracellular production of PHA and TAG in a ratio of between 20:80 and 80:20 of PHA:TAG. In this regard, the method of the invention comprises the enrichment of the culture in microorganisms capable of hydrolysing the substrate and accumulating TAG, but also in microorganisms capable of accumulating PHA. In particular, the method comprises a feeding of lipidic substrate in pulses (feast phase) uncoupled from the nitrogen source (which is added at the end of the feast and consumed in the famine). Excess carbon must be limited to ensure that the culture is partially enriched in PHA accumulators. To that end, in a preferred embodiment, during the feast phase, each feeding pulse contains an amount of carbon equal to or less than 1,200 mg COD. In another more preferred embodiment, a pulse is added when 85% of the carbon from the previous pulse has been consumed or depleted, which leaves 15% of carbon still unconsumed, which would correspond to the non-biodegradable fraction of the carbon source. Carbon consumption or depletion can be monitored by the amount of dissolved oxygen (DO). In another preferred embodiment, the amount of carbon at the beginning of the famine phase will be equal to or less than 15%. Furthermore, consumption of the added nitrogen for microbial growth during the famine phase must be ensured so that the next operation cycle begins under nitrogen-limiting conditions, in other words, less than 20 mg TN/L.

A particular embodiment that illustrates the invention is described below.

### Methods and experimental design

The process was carried out in an SBR-type reactor operated on a laboratory scale. The reaction vessel (1, see Figure 1) was made of methacrylate and had an external coil for temperature control, its working volume was 10 L (total volume 15 L). Furthermore, the system had: 1 a fixed flow peristaltic pump for feeding the lipidic stream used as a carbon source (2); 2 variable flow peristaltic pumps and their corresponding controllers to carry out volume exchange in the system (pump for the discharge of 50% of the reaction volume, and feeding pump for the aqueous stream to fill the reactor, (3) and (4), respectively); 1 a blower to supply air to the reaction medium (5); thermostatted bath for temperature control (6); oxygen probe for continuous monitoring of dissolved oxygen (DO) and temperature (HQ40d HACH Lange, USA) (7); programmable logic controller (PLC) Siemens S7-1200 for programming the operation cycle (actuating the pumps at the desired times) (8); 1 L vessel for the lipidic source (9); 20 L bag for the aqueous stream (10); and 30 L container for collecting the discharge (11).

For its implementation, the reactor was inoculated at the beginning of the process with 10 L of activated sludge from the Calo-Milladoiro wastewater treatment plant (A Coruña). These sludges had an initial concentration of total and volatile suspended solids (TSS and VSS, respectively) of 3.63 ± 0.16 g TSS/L and 3.01 ± 0.17 g TSS/L and a pH of 7.5.

The reactor was continuously aerated and completely mixed due to a pair of diffusers located in the lower part of the reactor through which air was introduced driven by an air pump. There was no mechanical stirring system. The temperature was controlled at 30 ÷ 2°C due to an exterior coil and a thermostatted bath. The pH of the medium was not controlled by an automated control system but was maintained around neutral (7.0 ± 0.3) throughout the entire operation cycle by adding a bicarbonate buffer to the aqueous stream.

In the activated sludge used as an inoculum, there were different microbial populations, from which it was necessary to select a culture with the ability to perform the following functions:
1) Hydrolysing the fats present in the lipidic stream used as a carbon source into long chain fatty acids and glycerol.
2) Intracellularly accumulating the compounds resulting from the hydrolysis process such as PHA and/or TAG.

To that end, the system was operated under the feast/famine regime considering the modification of the conventional strategy (ADF) that involves feeding the carbon and nitrogen sources in a decoupled manner, in other words, the DGL strategy (Oliveira, et al., N.Biotechnol. 2017, 37, 69-79). In this regard, the lipidic stream (carbon source) was added at the beginning of the cycle and in the absence of nitrogen, giving rise to the feast phase and once completed, the nitrogen source was added in the absence of carbon, giving rise to the famine phase. The addition of the carbon source took place in pulses throughout the entire feast phase.

Three different process configurations were tested consisting of operation cycles of 12 hours, 18 hours and 24 hours, as shown in Figure 3, distributed as follows: (I) Feast phase (360 - 720 minutes): carbon source feeding intervals (2 minutes) + aerobic reaction (58-118 minutes); (II) exchange of 50% of the reaction volume (emptying + filling); and (III) famine phase (348 - 708 minutes), aerobic reaction after adding the nitrogen source (348 - 708 minutes).

*Feast phase:* the carbon source consisted of the fat fraction of an effluent of the canning industry from tuna production with a high oleic acid content (Table 1).

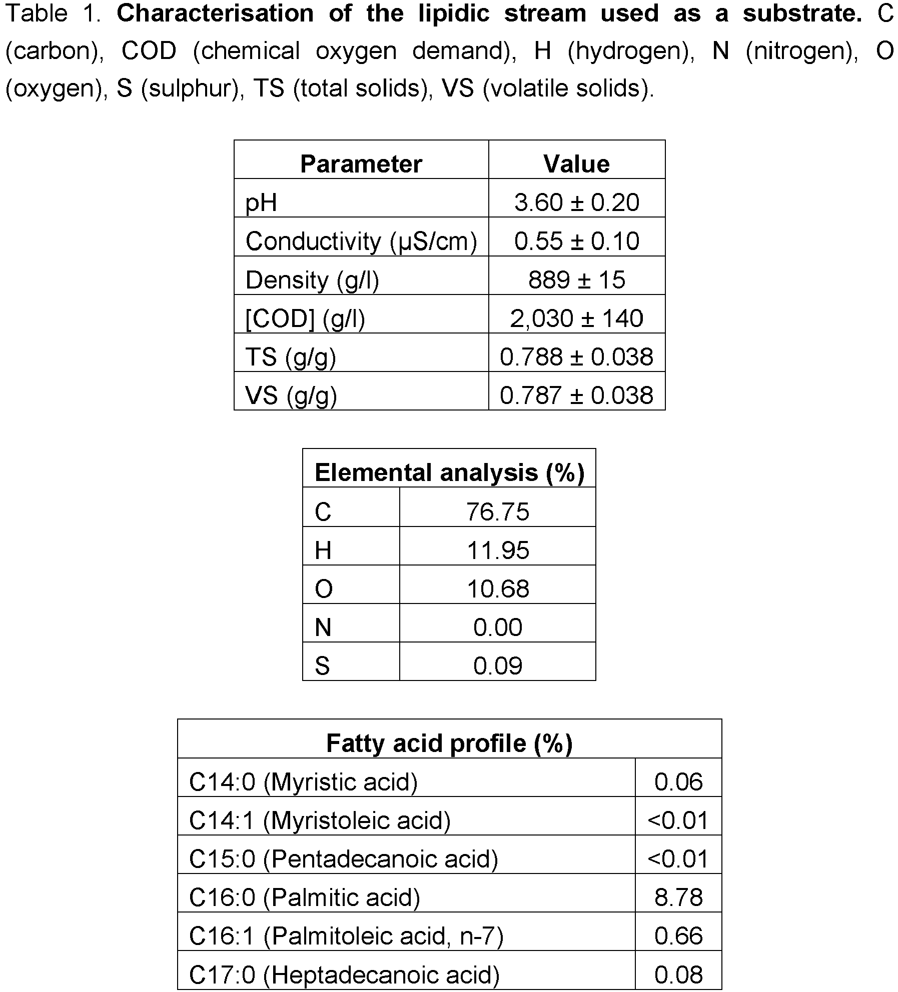

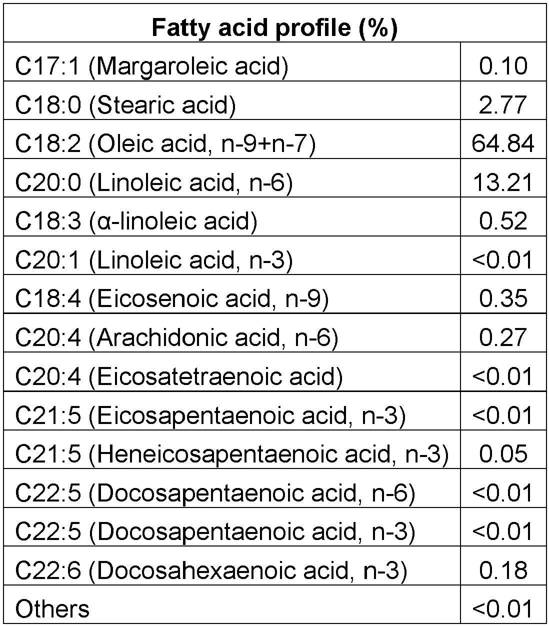

This lipidic fraction from an effluent of the canning industry was added in pulses (with a frequency of between 1 and 2 hours) from the beginning of the cycle to the end of the feast phase. Each time a substrate pulse was consumed, a new one was added. The periodicity between pulses was set as a function of the DO concentration measured continuously in the reactor using an oxygen probe.
- When the carbon source was added, the DO concentration decreased as a consequence of the metabolisation of the substrate, and subsequently rose until reaching saturation values again, which indicated the depletion of the carbon source.
- The time elapsed between the addition of the carbon pulse, and the moment in which saturation values were reached again defined the time necessary for the consumption of the substrate. In other words, the periodicity between pulses.
- Once said time is defined, the moment at which each pulse was to be added was programmed in a programmable logic controller (PLC).
- The evolution of the DO concentration was recorded during continuous monitoring and the profiles were reviewed daily in case the substrate consumption time varied and readjustments had to be made in the PLC programming.

*Exchange:* At the end of the feast phase, half of the reactor volume (5 L containing biomass with accumulated PHA and TAG) was removed for extraction and purification of the biopolymers. Subsequently, the same volume (5 L) of an aqueous stream that consisted of a nutrient solution (nitrogen source) was added, starting the famine phase.

*Famine phase:* The nitrogen source used consisted of a synthetic solution that contained the minimum amount of nitrogen in the form of NH₄Cl required for the duplication of the microorganisms present in the reaction medium (growth), NaHCOs buffer to maintain the pH of the system around neutral, allylthiourea to inhibit nitrifying activity and other macro- and micronutrients (aqueous stream, Table 2).

**Table 2. Composition of the aqueous stream containing the nitrogen source.**

| **Compound** | **Value** |
|---|---|
| NH₄Cl (mg/l) | 10.00 - 250.00 |
| KH₂PO₄ (mg/l) | 25.00 - 300.00 |
| NaHCO₃ (mg/l) | 55.00 - 396.00 |
| MgSO₄ (mg/l) | 9.90 |
| KCl (mg/l) | 66.00 |
| Allylthiourea (mg/l) | 4.36 |
| Solution of trace elements (ml/L) * | 1.00 |

| | |
|---|---|
| * Vishniac, W., Santer, M., 1957. The thiobacilli. Bacteriol. Rev. 21, 195-213 | |

The minimum amount of nitrogen necessary for microbial growth, or, in other words, the stoichiometric amount of nitrogen for microbial growth, is determined as follows:
- An empirical formula for biomass is estimated based on the bibliography (Popovic, Heliyon, 2019, 5, 6) (C_{X}H_{Y}N_{W}O_{Z}) considering the type of microbial populations existing in the system (PHA and TAG accumulators). Once the formula of the biomass is defined, the moles of nitrogen necessary to form one mole of biomass are known. Therefore, if the moles of biomass existing in the reactor are known, it is possible to calculate the moles of nitrogen necessary for the doubling of biomass by stoichiometry. That is:
   - According to the formula of the biomass CₐH_{b}N_{c}O_{d}, to form 1 mole of biomass, c moles of nitrogen are needed.
   - If M are the moles of active biomass in the reactor, .2 × M × c moles of nitrogen are needed for the biomass to be doubled.
   In the examples shown here, the following was considered as the elemental formula of the biomass: CH_{1.8}O_{0.5}N_{0.2}. Therefore, to form 1 mole of biomass, 0.2 moles of nitrogen were needed.
- Subsequently, the actual amount of nitrogen that microorganisms need for growth in each cycle is readjusted based on the concentration of nitrogen remaining at the end of the famine phase, where it should be zero. To know its value, the concentration of total nitrogen present in the liquid phase of a sample taken in the reaction medium at the end of the famine phase is analysed (see sampling and analysis section).

### Sampling and analysis

During the characterisation of the operation cycles of the SBR, periodic samples of the reaction volume were taken. In the feast phase, these were taken immediately before the addition of each pulse of carbon source.

The pH was determined with a pH & Ion meter (GLP 22 Crison, Spain). The concentrations of total suspended solids (TSS), volatile suspended solids (VSS), total chemical oxygen demand (CODt), and soluble chemical oxygen demand (CODₛ) were analysed according to the Standard Methods for the Examination of Water and Wastewater (Baird, R., & Bridgewater, L. 2017.Standard methods for the examination of water and wastewater. 23rd edition. Washington, D.C.: American Public Health Association; Methods 2540 E pages 4-6 (TSS,VSS), and 5220 B pages 2-3 (COD)). The ion concentration was determined by ion chromatography (861 Advanced Compact IC Metrohm, Switzerland), and the total organic carbon, inorganic carbon and total nitrogen (TOC, IC, and TN, respectively) by catalytic combustion (TOL-L analyser with the TNM module, TOC-5000 Shimazdu, Japan). The CODₛ, ion, TOC, IC, and TN concentrations were determined in the soluble fraction of the sample after centrifugation (5430 Eppendorf centrifuge, USA) and filtration (filters of 0.45 µm pore size with a cellulose-ester membrane, Advantec, Japan) of the raw sample. To characterise the carbon source, the conductivity was measured with a portable conductivity meter (probe Sension + EC5 HACH-Lange, USA); the concentration of total and volatile solids (TS and VS, respectively) as described in the Standard Methods for the Examination of Water and Wastewater (Baird, R., & Bridgewater, L. 2017.Standard methods for the examination of water and wastewater. 23rd edition. Washington, D.C.: American Public Health Association; Methods 2540 B p. 2 (TS), and 2540 D p. 6 (VS)); its elemental composition using an elemental analyser (FlashEA 1112 Thermo Scientific, USA); and the fatty acid profile by gas chromatography according to ISO standard 12966-2:2011 (4.2) - rapid method, and ISO standard 12966-4:2015.

The PHA and TAG accumulated intracellularly in the biomass were determined by gas chromatography (innovax HP column equipped with a flame ionisation detector (FID) (Agilent, USA)) following the method described by Smolders et al. Biotechnol. Bioeng. 1994, 44, 837-848. To do so, samples of the reaction volume were taken, centrifuged (5420 Eppendorf Centrifuge, USA), frozen and lyophilised to obtain a solid phase. The accumulated compounds were quantified using commercial calibration standards for PHA (copolymer containing 88% hydroxybutyrate (HB), and 12% hydroxyvalerate (HV)) and TAG (palmitic acid, stearic acid, oleic acid and linoleic acid) (Sigma Aldrich, USA).

### Calculations

The content of intracellular compounds (PHA and TAG) contained in the biomass was expressed as a percentage of dry weight of the cell (d.w. %), as a percentage of the measured VSSs. The active biomass concentration (X) was calculated as the difference between the mass of the VSSs and the sum of the accumulated intracellular compounds (Biop = PHA + TAG) considering the following empirical formula for the CH_{1.8}O_{0.5}N_{0.2} biomass.

The maximum consumption of carbon and nitrogen (-qs, -q_{N}, respectively), and maximum production rates of PHA, TAG and active biomass (q_{PHA}, q_{TAG}, q_{X}, respectively), were determined based on the maximum slopes of the curves that describe the evolution of the concentration of the different parameters over time. These parameters were expressed as Cmmol/(Cmmolₓ·h) except qₓ, which referred to the substrate (S) and was defined as Cmmol/(Cmmols h). Production yields (Y, expressed as Cmmol/Cmmols) were determined by dividing the maximum production rates by the maximum consumption rate of S. The maximum daily productivity refers to the mass (in g) of accumulated intracellular compounds (PHA and TAG) daily per volume (in L) of substrate fed to the system. This value was determined by dividing the yield (previously transforming the Cmmol values to g) by the litres of lipidic stream fed to the system per day.

Three specific examples relating to the operation of the system under the different cycle configurations tested are shown below.

### Example 1.

The reactor described above was operated in 12-hour cycles for 57 days. The cycle exemplified here corresponds to day 43 of operation.

The carbon source (lipidic stream) was added in pulses during the feast phase (first 6 hours). A total of 6 pulses of 0.6 ml (1.20 g COD/pulse, taking into account that the lipidic stream contained 2,000 g COD/L) with a frequency of 1 hours between pulses (2 minutes of feeding the carbon source + 58 minutes of aerobic reaction), which meant a flow of 3.42 Cmmol/(L.h) during this first phase.

Subsequently, 50% of the reaction volume (5 L) was removed, and 5 L of an aqueous stream with a nitrogen concentration of about 16 mg NTZL was added.

The famine phase took place during the remaining 6 hours.

Figure 4 (a) shows the evolution of the main operating parameters monitored in the reactor. Table 3 shows the intracellular accumulation values, the composition of the accumulated compounds, the main kinetic parameters, and the yields achieved. After 6 hours (end of the feast phase), an intracellular accumulation of 50.45 ± 0.22 d.w. was reached. % (ratio PHA:TAG 50.5:49.5). At this point, the maximum accumulation rates of PHA and TAG were 0.088 Cmmol_{PHA}/(Cmmol_{S}·h) and 0.073 Cmmol_{TAG}/(Cmmol_{S}·h), respectively, with a consumption rate of the lipidic substrate of -0.458 Cmmol_{S}/Cmmol_{X}·h). The production yields of PHA and TAG were 0.231 Cmmol_{PHA}/Cmmol_{S} and 0.192 Cmmol_{TAG}/Cmmol_{S}, which resulted in a total yield of 0.423 Cmmol_{Biop}/Cmmol_{S}.

**Table 3. Intracellular accumulation, kinetic parameters and yields obtained in the SBR reactor on day 43 of operation (12-hour cycle).**

| **Parameter** | **Phase of the cycle** | **Values** |
|---|---|---|
| Carbon stream (Cmmol/(L·h) | Feast | 3.42 |
| Intracellular accumulation (d.w. %) | Final feast | 50.24 ± 0.44 |
| PHB:PHV:PAL:STE:OL:LIN | Final feast | 46:3:6:1:32:12 |
| q_{TAG} (Cmmol_{TAG}/Cmmol_{X}·h) | Feast | 0.073 |
| q_{PHA} (Cmmol_{PHA}/Cmmol_{X}·h) | Feast | 0.088 |
| q_{Biop} (Cmmol_{PHA}/Cmmol_{X}·h) | Feast | 0.162 |
| - q_{N} (Cmmol_{N}/Cmmol_{X}·h) | Feast/Famine | -0.001 / -0.012 |
| - q_{C} (Cmmol_{C}/Cmmol_{X}·h) | Feast | -0.458 |
| q_{X} (Cmmol_{X}/Cmmol_{C}·h) | Feast/Famine | 0.002 / 0.003 |
| Y_{TAG} (Cmmol_{TAG}/Cmmol_{C}) | Feast | 0.192 |
| Y_{PHA} (Cmmol_{PHA}/Cmmol_{C}) | Feast | 0.231 |
| Y_{Biop} (Cmmol_{PHA}/Cmmol_{C}) | Feast | 0.423 |
| Y_{X} (Cmmol_{X}/Cmmol_{S}) | Feast/Famine | 0.023 / 0.049 |

| | | |
|---|---|---|
| Biop (PHA + TAG accumulated intracellularly); C (lipidic substrate); LIN (linoleic acid); OL (oleic acid); PAL (palmitic acid), PHA (polyhydroxyalkanoate); PHB (polyhydroxybutyrate); PHV (polyhydroxyvalerate); q (specific maximum production); - q (specific maximum consumption); STE (stearic acid); TAG (triacylglyceride); X (active biomass); Y (performance). | | |

### Example 2.

The reactor described above was operated, starting on day 58 of operation, in 18-hour cycles. At this point, the duration of the feast phase was increased from 6 to 12 hours, while the famine phase remained at 6 hours. The example considered here corresponds to day 72 of operation.

The carbon source (lipidic stream) was added in pulses during the feast phase (first 12 hours). A total of 6 pulses of 0.6 ml (1.20 g COD/pulse, taking into account that the lipidic stream contained 2,000 g COD/L) with a frequency of 1 hour between pulses (2 minutes of feeding the carbon source + 118 minutes of aerobic reaction), which meant a flow of 1.71 Cmmol/(L.h) during this first phase.

Subsequently, 50% of the reaction volume (5 L) was removed, and 5 L of an aqueous stream with a nitrogen concentration of about 18 mg TN/L was added.

The famine phase took place during the remaining 6 hours.

Figure 4 (b) shows the evolution of the main operating parameters monitored in the reactor. Table 4 shows the intracellular accumulation values, the composition of the accumulated compounds, the main kinetic parameters, and the yields achieved. The rates of carbon and nitrogen consumption during the feast and famine phases, respectively, were lower than in the 12-hour cycle (Example 1), which resulted in lower carbon and nitrogen consumption during each of the phases. Therefore, nitrogen was in a higher proportion in the reaction medium (6 vs. 13 mg/l at the end of the feast phase in the 12- and 18-hour cycles, respectively), and there was also a greater amount of extracellular carbon during the famine phase (80 vs. 400 mg COD/L). Therefore, nitrogen was under less limiting conditions during the feast phase and there was a high amount of carbon during the famine phase (> 10 - 15% of the COD added to the reaction medium during the feast phase, which is a percentage corresponding to the non-biodegradable COD of the substrate) allowing the growth of non-accumulator populations. Consequently, a reduction in the accumulation capacity of the system was observed. Between days 43 (Example 1) and 72 (Example 3), the maximum intracellular accumulation decreased from 50.24 ± 0.44 (TAG:PHA = 51:49) to 30.75 ± 1.67 (TAG:PHA = 65:35) dry weight % (d.w. %) and the maximum production yield of intracellular compounds from 0.429 to 0.299 Cmmol_{Biop}/Cmmol_{S}.

**Table 4. Intracellular accumulation, kinetic parameters and yields obtained in the SBR reactor on day 72 of operation (18-hour cycle).**

| **Parameter** | **Phase of the cycle** | **Values** |
|---|---|---|
| Carbon stream (Cmmol/(L·h) | Feast | 1.71 |
| Intracellular accumulation (d.w. %) | Final feast | 30.75 ± 1.67 |
| PHB:PHV:PAL:STE:OL:LIN | Final feast | 9:26:9:2:37:17 |
| q_{TAG} (Cmmol_{TAG}/Cmmol_{X}·h) | Feast | 0.020 |
| q_{PHA} (Cmmol_{PHA}/Cmmol_{X}·h) | Feast | 0.012 |
| q_{Biop} (Cmmol_{PHA}/Cmmol_{X}·h) | Feast | 0.032 |
| - q_{N} (Cmmol_{N}/Cmmol_{X}·h) | Feast/Famine | -0.006 / -0.010 |
| - q_{C} (Cmmol_{C}/Cmmol_{X}·h) | Feast | -0.069 |
| q_{X} (Cmmol_{X}/Cmmol_{C}·h) | Feast/Famine | 0.006 / 0.064 |
| Y_{TAG} (Cmmol_{TAG}/Cmmol_{C}) | Feast | 0.186 |
| Y_{PHA} (Cmmol_{PHA}/Cmmol_{C}) | Feast | 0.113 |
| Y_{Biop} (Cmmol_{PHA}/Cmmol_{C}) | Feast | 0.299 |
| Y_{X} (Cmmol_{X}/Cmmol_{S}) | Feast/Famine | 0.425 / 0.390 |

| | | |
|---|---|---|
| Biop (PHA + TAG accumulated intracellularly); C (lipidic substrate); LIN (linoleic acid); OL (oleic acid); PAL (palmitic acid); PHA (polyhydroxyalkanoate); PHB (polyhydroxybutyrate); PHV (polyhydroxyvalerate); q (specific maximum production); - q (specific maximum consumption); STE (stearic acid); TAG (triacylglyceride); X (active biomass); Y (performance). | | |

### Example 3.

The reactor described above was operated, starting on day 96 of operation in 24-hour cycles. In the present example, a cycle corresponding to day 133 of operation is analysed.

To ensure greater consumption of carbon and nitrogen sources during feast and famine phases, respectively, the configuration of the operation cycle was modified compared to that described in Example 2. To that end, the feast phase was maintained at 12 hours, but to avoid the presence of extracellular carbon during the famine phase, the amount of substrate added during this phase was reduced. To do so, a total of 5 pulses of 0.60 ml each, (1.20 g COD/pulse, taking into account that the lipidic stream contained 2,000 g COD/L) were added instead of 6 pulses of 0.6 ml, which meant a flow of 1.45 Cmmol/(L.h). The MMC was fed with the carbon source as follows: 4 intervals of 2 minutes of feeding the carbon source + 118 minutes of aerobic reaction; plus a final interval of 2 minutes of feeding the carbon source + 236 minutes of aerobic reaction.

Subsequently, 50% of the reaction volume (5 L) was removed, and 5 L of an aqueous stream with a nitrogen concentration of around 10 mg/L was added.

The famine phase took place during the remaining 12 hours. The duration of the famine phase was extended from 6 to 12 hours compared to Example 2, and the amount of nitrogen added was readjusted, based on the concentration of total nitrogen (TN) measured at the end of the operation cycle, to ensure more limiting nitrogen conditions than in Example 2.

In this way it was possible to reduce the amount of extracellular carbon available in the reaction medium at the end of the feast phase (50 vs. 400 mg COD/L) and further limit the concentration of nitrogen available in the reaction medium at the end of the famine phase (5 vs. 13 mg TN/L), which had a positive impact on the accumulation capacity of the culture compared to the 18-hour cycle.

Figure 4 (c) shows the evolution of the main operating parameters monitored in the reactor. Table 5 shows the intracellular accumulation values, the composition of the accumulated compounds, the main kinetic parameters, and the yields achieved. Between days 72 (Example 2) and 133 (Example 3) of operation, intracellular accumulation at the end of the feast phase (12 hours) increased from 30.75 ± 1.67 (TAG:PHA = 65:35) to 45.31 ± 2.18 (TAG:PHA = 25:75) d.w. % despite adding a smaller amount of carbon source. Consequently, an increase in the production yield of intracellular compounds of 96% (from 0.299 to 0.586 Cmmol_{Biop}/Cmmol_{S}) was observed.

**Table 5. Intracellular accumulation, kinetic parameters and yields obtained in the SBR reactor on day 133 of operation (24-hour cycle).**

| **Parameter** | **Phase of the cycle** | **Values** |
|---|---|---|
| Carbon stream (Cmmol/(L·h) | Feast | 1.45 |
| Intracellular accumulation (d.w. %) | Final feast | 45.31 ± 2.18 |
| PHB:PHV:PAL:STE:OL:LIN | Final feast | 8:17:8:1:48:18 |
| q_{TAG} (Cmmol_{TAG}/Cmmol_{X}·h) | Feast | 0.083 |
| q_{PHA} (Cmmol_{PHA}/Cmmol_{X}·h) | Feast | 0.016 |
| q_{Biop} (Cmmol_{PHA}/Cmmol_{X}·h) | Feast | 0.099 |
| - q_{N} (Cmmol_{N}/Cmmol_{X}·h) | Feast/Famine | -0.001 / -0.005 |
| - q_{C} (Cmmol_{C}/Cmmol_{X}·h) | Feast | -0.164 |
| q_{X} (Cmmol_{X}/Cmmol_{C}·h) | Feast/Famine | 0.008 / 0.0105 |
| Y_{TAG} (Cmmol_{TAG}/Cmmol_{C}) | Feast | 0.489 |
| Y_{PHA} (Cmmol_{PHA}/Cmmol_{C}) | Feast | 0.097 |
| Y_{Biop} (Cmmol_{PHA}/Cmmol_{C}) | Feast | 0.586 |
| Y_{X} (Cmmol_{X}/Cmmol_{S}) | Feast/Famine | 0.086 / 0.478 |

| | | |
|---|---|---|
| Biop (PHA + TAG accumulated intracellularly); C (lipidic substrate); LIN (linoleic acid); OL (oleic acid); PAL (palmitic acid); PHA (polyhydroxyalkanoate); PHB (polyhydroxybutyrate); PHV (polyhydroxyvalerate); q (specific maximum production); - q (specific maximum consumption); STE (stearic acid); TAG (triacylglyceride); X (active biomass); Y (performance). | | |

### Comparison with the literature

Table 6 shows a comparison of the results obtained in the present invention (Example 1), with three representative references from the literature in which a mixed culture is used for: (a) accumulation of PHA from a lipidic stream; (b) accumulation of TAG from a lipidic stream; and (c) preferential accumulation of PHA or TAG from a lipidic stream.

**Table 6. Comparison of the results obtained in the present invention with the literature.**

| | **Feeding** | **Configuration of the system** | **Maximum accumulation (% d.w.)** | **Productivity (g Biop/(L·d)** | **Reference** |
|---|---|---|---|---|---|
| a) | Waste water from olive oil production | Pretreatment + SBR + FBR | 30.00% PHA | 1.50 | Campanari et al. (2014) |
| b) | Soy bean plant oil | SBR + FBR | 54.00 d.w. % TAG | - | Tamis et al. (2015) |
| c) | Waste lipidic stream from cooking tuna | SBR + FBR | 82.30 PHA + 3.33 TAG | 0.88 0.67 | Argiz et al., (2021) |
| | | | 5.80 PHA + 39.55 TAG | | |
| d) | Waste lipidic stream from cooking tuna | SBR | 24.62 PHA + 25.48 TAG | 1.21 | Present invention, at 6 hours of operation |

| | | | | | |
|---|---|---|---|---|---|
| Biop (sum of PHA and TAG), FBR *(fed batch reactor),* PHA (polyhydroxyalkanoate), SBR *(sequencing batch reactor).* | | | | | |

In a previous operation (Argiz et al., Sci. Total Environ. 2021, 763, 142944), in which the same carbon source was used in a two-unit process (SBR enrichment reactor + FBR accumulation reactor), 12 hours of operation were needed in the SBR and 30 hours in the FBR to achieve an accumulation of 82.30% PHA by dry weight and 3.33% TAG by dry weight (1.8 g VSS/L, 0.45 g X/L) when the PHAs were the main accumulated compounds. When TAG accumulation was preferential, 12 hours of the operation cycle of the SBR and an additional 27 hours in the FBR were required to achieve an accumulation of 39.55% TAG by dry weight and 5.80% PHA by dry weight (2.1 g VSS/L, 1.1 g X/L). Therefore, maximum productions of 0.88 and 0.67 g Biop/(L·d) respectively were reached, while in the present invention, with the single-unit process, despite the fact that the biomass concentration was lower (0.48 g VSS/L, 0.22 g X/L) a maximum of 1.21 g Biop/(L·d) was reached.

## Claims

1. A process for the intracellular production of polyhydroxyalkanoates (PHA) and triacylglycerides (TAG) in a mixed microbial culture, **characterised in that** all the steps are carried out in a single sequencing batch reactor, and which further comprises:
a) inoculating activated sludge in the reactor,
b) an operation cycle of the reactor, comprising:
i. feeding the microorganisms present in the reactor with a lipidic carbon source, the feeding being in pulses and under limiting nitrogen conditions
ii. removing 50% of the mixture from the reactor, wherein said mixture contains the biomass with intracellular PHA and TAG,
iii. adding a stream containing between 0.9 and 2 times the stoichiometric amount of nitrogen necessary for microbial growth,
c) repeating the process from step b) in successive cycles.

2. The process according to claim 1, wherein the activated sludge comprises PHA- and TAG-accumulating microorganisms, and microorganisms capable of hydrolysing lipids.

3. The process according to any of the preceding claims, wherein the activated sludge comprises PHA-accumulating microorganisms selected from among *Acidovorax, Acinetobacter, Pandoraea, Azospirillum,* and *Pseudomonas;* and TAG-accumulating microorganisms selected from among *Candida, Yarrowia, Geotrichum, Mortierella, and Rhodoccocus,* and microorganisms capable of hydrolysing lipids selected from *Candida, Yarrowia, Fonsecaea, Capronia, Acinetobacter, Cryptococcus, Rhodosporidium, Geotrichum* and *Trichosporon.*

4. The process according to any of the preceding claims, wherein the activated sludge comes from an urban Wastewater Treatment Plant (WWTP).

5. The process according to any of the preceding claims, wherein the lipidic carbon source has a lipid content greater than 85% of the chemical oxygen demand (COD).

6. The process according to any of the preceding claims, wherein each feeding pulse contains an amount of carbon equal to or less than 1,200 mg COD.

7. The process according to any of the preceding claims, wherein the amount of nitrogen in step (i) is less than 20 mg total nitrogen (TN)/L.

8. The process according to any of the preceding claims, wherein the amount of nitrogen in step (i) is between 5 and 20 mg TN/L.

9. The process according to any of the preceding claims, wherein step ii) takes place immediately after step i).

10. The process according to any of the preceding claims, wherein step (i) lasts between 6 hours and 12 hours.

11. The process according to any of the preceding claims, wherein step (iii) lasts between 6 and 12 hours.

12. The process according to any of the preceding claims, wherein the stream of step (iii) is an aqueous solution with a nitrogen compound.

13. The process according to claim 12, wherein the nitrogen compound is selected from ammonium sulphate, ammonium nitrate, and ammonium chloride.

14. The process according to claim 12, wherein the aqueous solution contains a concentration of between 5 mg/l and 280 mg/l of ammonium chloride.

15. The process according to any of the preceding claims, wherein at the end of phase (iii) the amount of total nitrogen in the reaction medium is less than 20 mg of total nitrogen per litre.
